# EUROPEAN PATENT APPLICATION

(11) **EP 3 730 604 A1**
(43) Date of publication of application: **28.10.2020**
(21) Application number: 19768664.5
(22) Date of filing: 15.03.2019
(51) Int. Cl.: C12N 5/071, A61L 27/38, C12N 5/077

(54) **METHOD FOR PRODUCING SHEET-LIKE CELL CULTURE**

(30) Priority: 15.03.2018 JP 2018047911
(71) Applicant: Terumo Kabushiki Kaisha, Tokyo 151-0072 (JP)
(72) Inventor: SAMESHIMA, Tadashi, Ashigarakami-gun, Kanagawa 259-0151 (JP); NOGUCHI, Eri, Ashigarakami-gun, Kanagawa 259-0151 (JP); KANNO, Tomonori, Ashigarakami-gun, Kanagawa 259-0151 (JP)
(74) Representative: Prüfer & Partner mbB Patentanwälte · Rechtsanwälte
(86) International application number: PCT/JP2019/010819
(87) International publication number: WO 2019/177148

(57) **Abstract**

An object of the present disclosure is to provide a high-quality sheet-shaped cell culture by preventing breakage of a sheet-shaped cell culture that is caused by adhesion, sticking, shrinkage, etc. of the sheet-shaped cell culture in a manipulation such as detaching, transfer, or preservation after the formation of the sheet-shaped cell culture. The issue mentioned above has been solved by methods of detaching, cleaning, and/or preservation including soaking a sheet-shaped cell culture in a low nutrient isotonic solution.

## Description

### Technical Field

The present disclosure relates to a method for preventing adhesion, sticking, shrinkage, etc. of a sheet-shaped cell culture when detaching, transferring, or preserving the sheet-shaped cell culture in the production of the sheet-shaped cell culture, etc.

### Background Art

In recent years, various cells are being transplanted in order to repair damaged tissues and the like. For example, the use of fetal myocardiac cells, skeletal myoblasts, mesenchymal stem cells, cardiac stem cells, ES cells, iPS cells and the like are being used to repair cardiac muscle tissues damaged, for example, by ischemic cardiac heart disease, such as angina pectoris and cardiac infarction, and the like (Non Patent Literature 1).

As part of such an attempt, a cell structure formed using a scaffold and a sheet-shaped cell culture (a cell sheet) in which cells are formed in a sheet shape have been developed (Patent Literature 1). However, a cell sheet is generally fragile and is likely to develop wrinkles, tears, etc. at the time of isolation from a culture substrate or during subsequent manipulations, and considerable skill has been necessary for manipulations such as transfer, preservation, and transplantation. In regard to such a problem, methods for suppressing wrinkles, folds, etc. of a sheet-shaped cell culture and methods for detecting them have been developed (Patent Literatures 2 to 5). However, all such wrinkles and folds occur due to detaching manipulation, handling during transfer, etc. of the sheet-shaped cell culture.

### Citation List

### Patent Literature

Patent Literature 1: JP 2007-528755 A
Patent Literature 2: JP 2016-052270 A
Patent Literature 3: JP 2012-029605 A
Patent Literature 4: JP 2011-115080 A
Patent Literature 5: JP 2012-152161 A

### Non Patent Literature

Non Patent Literature 1: Haraguchi et al., Stem Cells, Transl Med. 2012; 1 (2): 136-41

### Summary of Invention

### Technical Problem

The present inventors have found out a problem that, even if wrinkles and folds physically occurring at the time of detaching or transfer are suppressed, apart from this, shrinkage or twists resulting from the physiological working of cells contained in the sheet-shaped cell culture occur and that, if such shrinkage or twists are left as they are, adhesion, sticking, etc. occur in this state, and the quality of the sheet-shaped cell culture is reduced.

Thus, an object of the present disclosure is to provide a high-quality sheet-shaped cell culture by suppressing shrinkage, twists, etc. of a sheet-shaped cell culture resulting from cellular physiological activities between cells, which conventional methods have yet to solve, and thereby preventing adhesion, sticking, tears, wrinkles, and breakage of the sheet-shaped cell culture.

### Solution to Problem

In the course of studying a sheet-shaped cell culture of skeletal myoblasts, the present inventors have found out new findings that a sheet-shaped cell culture of skeletal myoblasts formed on a culture substrate is allowed to have few twists or little shrinkage and also experience less adhesion or sticking by soaking the sheet-shaped cell culture in a low nutrient isotonic solution when detaching, transferring, and preserving the sheet-shaped cell culture. The present inventors further continued to conduct extensive studies on the basis of such findings, and have completed the present disclosure.

Described specifically, the present disclosure provides the followings.
[1] A method for suppressing shrinkage when detaching a sheet-shaped cell culture formed on a culture substrate, the method including:
   (a) soaking, in a low nutrient isotonic solution, an upper surface of the sheet-shaped cell culture of which at least part is adhered to the culture substrate; and
   (b) detaching the sheet-shaped cell culture in the low nutrient isotonic solution.
[2] The method according to [1], in which the culture substrate is covered with a serum.
[3] The method according to [1] or [2], in which the culture substrate is covered with a temperature-responsive material.
[4] The method according to any one of [1] to [3], in which the sheet-shaped cell culture contains a myoblast cell.
[5] The method according to any one of [1] to [4], in which the low nutrient isotonic solution is at 2 to 8°C.
[6] The method according to any one of [1] to [5], in which the low nutrient isotonic solution is Hanks' balanced salt solution.
[7] The method according to any one of [1] to [6], in which a production step-derived impurity remaining in the sheet-shaped cell culture is removed by the soaking.
[8] The method according to [1] to [7], further including extending the detached sheet-shaped cell culture.
[9] The method according to [8], in which the extending of the sheet-shaped cell culture is performed by shaking a container containing the low nutrient isotonic solution in which the sheet-shaped cell culture is soaked.
[10] A method for suppressing shrinkage of a sheet-shaped cell culture detached from a culture substrate, the method including perfusing the detached sheet-shaped cell culture with a low nutrient isotonic solution.
[11] The method according to [10], in which the perfusion is performed on both surfaces of the detached sheet-shaped cell culture.
[12] The method according to [10] or [11], in which the perfusion is performed by soaking the detached sheet-shaped cell culture in the low nutrient isotonic solution.
[13] The method according to [12], in which a procedure of removing the low nutrient isotonic solution in which the sheet-shaped cell culture is soaked, putting in a new low nutrient isotonic solution, and soaking the sheet-shaped cell culture again is performed at least once.
[14] The method according to any one of [10] to [13], in which the perfusion is performed during a period from immediately after the sheet-shaped cell culture is detached to when 6 hours have elapsed from the detaching.
[15] The method according to any one of [10] to [14], further including extending the sheet-shaped cell culture.
[16] The method according to [15], in which the extending of the sheet-shaped cell culture is performed by shaking a container containing the low nutrient isotonic solution in which the sheet-shaped cell culture is soaked.
[17] The method according to any one of [10] to [16], further including preserving the sheet-shaped cell culture while keeping the sheet-shaped cell culture soaked in a low nutrient isotonic solution.
[18] The method according to any one of [10] to [17], in which the sheet-shaped cell culture contains a myoblast cell.
[19] The method according to any one of [10] to [18], in which the low nutrient isotonic solution is at 2 to 8°C.
[20] The method according to any one of [10] to [19], in which the low nutrient isotonic solution is Hanks' balanced salt solution.
[21] A method for suppressing shrinkage when preserving a sheet-shaped cell culture detached from a culture substrate, the method including soaking the sheet-shaped cell culture in a low nutrient isotonic solution.
[22] The method according to [21], in which the soaking is performed when or after 6 hours have elapsed from detaching of the sheet-shaped cell culture.
[23] The method according to [21] or [22], further including extending the sheet-shaped cell culture.
[24] The method according to [23], in which the extending of the sheet-shaped cell culture is performed by shaking a container containing the low nutrient isotonic solution in which the sheet-shaped cell culture is soaked.
[25] The method according to any one of [21] to [24], in which the sheet-shaped cell culture contains a myoblast cell.
[26] The method according to any one of [21] to [25], in which the low nutrient isotonic solution is at normal temperature.
[27] The method according to any one of [21] to [26], in which the low nutrient isotonic solution is at 2 to 8°C at a time of a start of soaking.
[28] The method according to any one of [21] to [27], in which the low nutrient isotonic solution is Hanks' balanced salt solution.
[29] A method for suppressing shrinkage of a sheet-shaped cell culture detached from a culture substrate, the method including perfusing the detached sheet-shaped cell culture with a low nutrient isotonic solution.
[30] The method according to [29], in which the perfusion is performed on both surfaces of the detached sheet-shaped cell culture.
[31] The method according to [29] or [30], in which the perfusion is performed by soaking the detached sheet-shaped cell culture in the low nutrient isotonic solution.
[32] The method according to [31], in which a procedure of removing the low nutrient isotonic solution in which the sheet-shaped cell culture is soaked, putting in a new low nutrient isotonic solution, and soaking the sheet-shaped cell culture again is performed at least once.
[33] The method according to any one of [29] to [32], in which the perfusion is performed during a period from immediately after the sheet-shaped cell culture is detached to when 6 hours have elapsed from the detaching.
[34] The method according to any one of [29] to [33], further including extending the sheet-shaped cell culture.
[35] The method according to [34], in which the extending of the sheet-shaped cell culture is performed by shaking a container containing the low nutrient isotonic solution in which the sheet-shaped cell culture is soaked.
[36] The method according to any one of [29] to [35], further including preserving the sheet-shaped cell culture while keeping the sheet-shaped cell culture soaked in a low nutrient isotonic solution.
[37] The method according to any one of [29] to [36], in which the sheet-shaped cell culture contains a myoblast cell.
[38] The method according to any one of [29] to [37], in which the low nutrient isotonic solution is at 2 to 8°C.
[39] The method according to any one of [29] to [38], in which the low nutrient isotonic solution is Hanks' balanced salt solution.
[40] A method for suppressing shrinkage when preserving a sheet-shaped cell culture detached from a culture substrate, the method including soaking the sheet-shaped cell culture in a low nutrient isotonic solution.
[41] The method according to [40], in which the soaking is performed when or after 6 hours have elapsed from detaching of the sheet-shaped cell culture.
[42] The method according to [40] or [41], further including extending the sheet-shaped cell culture.
[43] The method according to [42], in which the extending of the sheet-shaped cell culture is performed by shaking a containing the low nutrient isotonic solution in which the sheet-shaped cell culture is soaked.
[44] The method according to any one of [40] to [43], in which the sheet-shaped cell culture contains a myoblast cell.
[45] The method according to any one of [40] to [44], in which the low nutrient isotonic solution is at normal temperature.
[46] The method according to any one of [40] to [45], in which the low nutrient isotonic solution is at 2 to 8°C at a time of a start of soaking.
[47] The method according to any one of [40] to [46], in which the low nutrient isotonic solution is Hanks' balanced salt solution.

### Advantageous Effects of Invention

By a method of the present disclosure, a risk that a sheet-shaped cell culture will experience adhesion, sticking, shrinkage, etc. due to a manipulation such as detaching, transfer, preservation, or transplantation after the sheet-shaped cell culture is formed is avoided, thereby tears, wrinkles, and breakage of the sheet-shaped cell culture are prevented, and manipulations can be performed easily. Thus, as a result, a sheet-shaped cell culture keeping high quality can be produced easily.

### Brief Description of Drawings

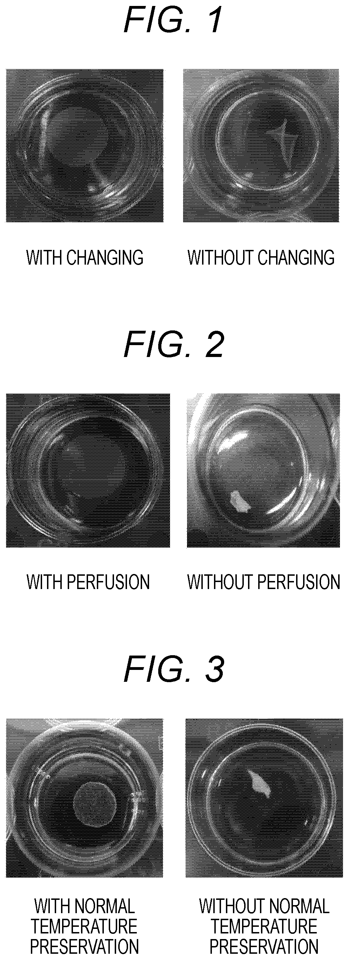

Fig. 1 is photographs showing a sheet-shaped cell culture preserved with changing of a culture medium of example 1 and a sheet-shaped cell culture preserved without changing of a culture medium. In the case where preservation was performed without changing, a twist and shrinkage of the sheet-shaped cell culture and adhesion resulting from these have been observed.
Fig. 2 is photographs showing sheet-shaped cell cultures in cases where preservation was performed with perfusion of a culture medium of example 2 and without perfusion. In the case where preservation was performed without perfusion, shrinkage of the sheet-shaped cell culture and adhesion resulting from it have been observed.
Fig. 3 is photographs showing sheet-shaped cell cultures in cases where preservation was performed with normal temperature preservation of example 3 and without normal temperature preservation. In the case where preservation was performed without normal temperature preservation, shrinkage of the sheet-shaped cell culture and adhesion resulting from it have been observed.

### Description of Embodiments

Hereinafter, the present disclosure will be explained in detail.

Unless otherwise defined herein, all the technical terms and scientific terms used in this description have the same meanings as generally understood by those skilled in the art. All the patents, applications, published applications and other publications referred to herein should be cited by reference in their entirety. In addition, if any contradiction arises between publications referred to in this description and recitations in this description, the recitations in this description supersede.

In the present disclosure, the term "sheet-shaped cell culture" means cells connected together into the shape of a sheet. Cells may be connected together directly (including those connected through via cellular elements, such as adhesion molecules) and/or connected together via an intervening substance. The intervening substance is not particularly limited insofar as it is a substance having at least an ability to physically (mechanically) connect cells together. Examples of the intervening substance may include extracellular matrices and the like. The intervening substance may preferably be a substance derived from cells, in particular, a substance derived from cells constituting the cell culture. Although the cells are connected at least physically (mechanically), the cells may be further connected functionally, for example, chemically or electrically. The sheet-shaped cell culture may be a culture configured with a single cell layer (single layer), or a culture configured with two or more cell layers (stacked (multilayer) architecture, for example, two layers, three layers, four layers, five layers, six layers, and so on). Further, in the sheet-shaped cell culture, cells may not exhibit a distinctive layer structure, but may have a three-dimensional structure having a thickness more than a thickness equivalent to one cell. For example, in a vertical cross section of the sheet-shaped cell culture, cells may not uniformly stand in a row in the horizontal direction, but may exist in a state of being arranged non-uniformly (for example, in a mosaic configuration).

It is preferred for the sheet-shaped cell culture to include no scaffold (support). A scaffold may be used in this technical field to maintain the physical integrity of a sheet-shaped cell culture by causing adhesion of cells on the surface and/or in the inside of the scaffold. Known scaffolds, for example, include a film made of polyvinylidene difluoride (PVDF) and the like. The sheet-shaped cell culture in the present disclosure can maintain the physical integrity of the sheet-shaped cell culture without such a scaffold. In addition, it may be preferred for the sheet-shaped cell culture in the present disclosure to consist of substances derived from cells constituting the sheet-shaped cell culture and to be free of other substances.

The cells constituting the sheet-shaped cell culture are not particularly limited, insofar as they can form the sheet-shaped cell culture, and examples include adhesion cells (adherent cells). Illustrative adhesion cells include adherent somatic cells (for example, myocardiac cells, fibroblast cells, epithelium cells, endothelial cells, hepatocytes, pancreatic cells, renal cells, adrenal gland cells, human periodontal ligament cells, gingiva cells, periosteum cells, skin cells, synovial cells, cartilage cells, and the like) and stem cells (for example, myoblast cells, tissue stem cells such as cardiac stem cells, embryonic stem cells, pluripotent stem cells, such as iPS (induced pluripotent stem) cells, mesenchymal stem cells and the like), and so on. The somatic cells may be stem cells, in particular, cells differentiated from iPS cells (adhesion cells derived from iPS cells). As for non-limiting examples of cells constituting the sheet-shaped cell culture, there are, for example, myoblast cells (for example, skeletal myoblasts and the like), mesenchymal stem cells (for example, bone marrow, adipose tissue, peripheral blood, skin, hair root, muscular tissue, endometrium, placenta, cord blood-derived cells, and the like), myocardiac cells, fibroblast cells, heart stem cells, embryonic stem cells, iPS cells, synovial cells, cartilage cells, epithelium cells (for example, oral mucosaepithelium cells, retinal pigment epithelial cells, nasal epithelial cells, and the like), endothelial cells (for example, vascular endothelial cells and the like), hepatocytes (for example, hepatic parenchymal cells and the like), pancreatic cells (for example, islet cells and the like), renal cells, adrenal gland cells, human periodontal ligament cells, gingiva cells, periosteum cells, skin cells and the like. As for non-limiting examples of the adhesion cells derived from iPS cells, there are myocardiac cells, fibroblast cells, epithelium cells, endothelial cells, hepatocytes, pancreatic cells, renal cells, adrenal gland cells, human periodontal ligament cells, gingiva cells, periosteum cells, skin cells, synovial cells, cartilage cells and the like, all of which have been derived from iPS cells.

In the present disclosure, a "myoblast cell" is a precursor cell of a striated muscle cell, and includes a skeletal myoblast and a cardiac myoblast.

In the present disclosure, a "skeletal myoblast" means a myoblast cell existing in a skeletal muscle. The skeletal myoblast is well known in the relevant technical field, and may be prepared from a skeletal muscle by an arbitrary known method (for example, a method described in JP 2007-89442 A, etc.) or may be obtained commercially (for example, Lonza, Cat# CC-2580). The skeletal myoblast can be identified by, for example, but not limited to, a marker such as CD56, α7 integrin, myosin heavy chain IIa, myosin heavy chain IIb, myosin heavy chain IId (IIx), MyoD, Myf5, Myf6, myogenin, desmin, or PAX3. In a certain embodiment, the skeletal myoblast is CD56-positive. In another certain embodiment, the skeletal myoblast is CD56-positive and desmin-positive. The skeletal myoblast is an arbitrary organism having a skeletal muscle, and may be derived from, for example, but not limited to, a mammal such as a human being, a nonhuman primate, a rodent (a mouse, a rat, a hamster, a guinea pig, etc.), a rabbit, a dog, a cat, a pig, a horse, a cow, a goat, or a sheep. In an embodiment, the skeletal myoblast is a skeletal myoblast of a mammal. In a certain embodiment, the skeletal myoblast is a human skeletal myoblast.

In the present disclosure, a "cardiac myoblast" means a myoblast cell existing in a cardiac muscle. The cardiac myoblast is well known in the relevant technical field, and can be identified by a marker such as Isl1. The cardiac myoblast may be derived from an arbitrary organism having a cardiac muscle, for example, but not limited to, a mammal such as a human being, a nonhuman primate, a rodent (a mouse, a rat, a hamster, a guinea pig, etc.), a rabbit, a dog, a cat, a pig, a horse, a cow, a goat, or a sheep. In an embodiment, the cardiac myoblast is a cardiac myoblast of a mammal. In a certain embodiment, the cardiac myoblast is a human cardiac myoblast.

In the present disclosure, a "myocardiac cell" means a cell having features of a myocardiac cell; examples of the features of a myocardiac cell include, but are not limited to, the expression of a myocardiac cell marker, the presence of autonomous pulsation, etc. Non-limited examples of the myocardiac cell marker include c-TNT (cardiac troponin T), CD172a (another name: SIRPA or SHPS-1), KDR (another name: CD309, FLK1, or VEGFR2), PDGFRA, EMILIN2, VCAM, and the like. Preferred examples of the myocardiac cell include a myocardiac cell derived from an iPS cell.

The cell contained in the sheet-shaped cell culture may be derived from an arbitrary organism that can undergo a medical treatment by means of the sheet-shaped cell culture. Examples of such an organism include, but are not limited to, a human being, a nonhuman primate, a dog, a cat, a pig, a horse, a goat, a sheep, an animal of the order Rodentia (for example, a mouse, a rat, a hamster, a guinea pig, etc.), a rabbit, and the like. The number of kinds of the cell contained in the sheet-shaped cell culture is not particularly limited, and the sheet-shaped cell culture may be composed only of cells of one kind or may use cells of two or more kinds. In the case where there are cells of two or more kinds forming the sheet-shaped cell culture, the content ratio (purity) of the largest number of cells is, at the time of the end of formation of the sheet-shaped cell culture, not less than 50%, preferably not less than 60%, more preferably not less than 70%, and still more preferably not less than 75%.

The cells may be xenogeneic derived cells or allogeneic derived cells. When the sheet-shaped cell culture is used for transplant, the term "the xenogeneic derived cells" as used herein means cells derived from a living organism different in species from the recipient. When the recipient is a human, for example, the xenogeneic derived cells can be cells derived from a monkey, a pig or the like. The term "allogeneic derived cells" means cells derived from a living organism of the same species as the recipient. When the recipient is a human, for example, the allogeneic derived cells can be human cells. The allogeneic derived cells include autologous derived cells (also referred to as host cells or autologous cells), in other words, recipient-derived cells, and also allogeneic non-host derived cells (also referred to as allogeneic cells). The autologous derived cells are preferred in the present disclosure, because their transplant causes no rejection reaction. However, xenogeneic derived cells and allogeneic non-host derived cells can be used. When xenogeneic derived cells or allogeneic non-host derived cells are used, an immunosuppression care may be required in order to inhibit a rejection reaction. It is to be noted that, in this description, cells other than autologous derived cells, that is, xenogeneic derived cells and allogeneic non-host derived cells may be collectively referred to as non-autologous derived cells. In an embodiment of the present disclosure, cells are autologous cells or allogeneic cells. In another embodiment of the present disclosure, cells are autologous cells. In a further embodiment of the present disclosure, cells are allogeneic cells.

A sheet-shaped cell culture can be produced by any known method (see, for example, Patent Literature 1, Patent Literature 2, JP 2010-081829 A, JP 2011-110368 A, or the like). A production method for producing a sheet-shaped cell culture typically includes a step of seeding cells on a culture substrate, a step of subjecting the seeded cells to sheet-forming culture, and a step of detaching the formed sheet-shaped cell culture from the culture substrate, but not particularly restricted. It is possible to conduct, before the step of seeding the cells on the culture substrate, a step of freezing the cells and a step of thawing the cells. Further, it is possible to perform a step of washing the cells, after the step of thawing the cells. Each of these steps can be implemented by any known method suited for producing the sheet-shaped cell culture. The production method of the present disclosure may include a step of producing the sheet-shaped cell culture. In this case, the step of producing the sheet-shaped cell culture may include, as a sub step on sub steps, one or two or more of the steps in the methods for producing the above-described sheet-shaped cell culture. In an embodiment, no step is included to proliferate cells after the step of thawing cells and before the step of seeding cells on a culture substrate.

The culture substrate is not particularly limited as long as it is one on which cells can form a cell culture, and examples include containers of various materials, a solid or semisolid surface in a container, and the like. The container is preferably formed of a structure and a material that do not allow a liquid such as a culture solution to permeate therethrough. Examples of such a material include, but are not limited to, polyethylene, polypropylene, Teflon (registered trademark), polyethylene terephthalate, polymethyl methacrylate, nylon 6,6, polyvinyl alcohol, cellulose, silicon, polystyrene, glass, polyacrylamide, polydimethylacrylamide, metals (for example, iron, stainless steel, aluminum, copper, and brass), etc. The container preferably has at least one flat surface. Examples of such a container include, but are not limited to, a culture container having a bottom surface formed of a culture substrate capable of forming a cell culture and a liquid-impermeable side surface. Certain examples of such a culture container include, but are not limited to, a cell culture dish, a cell culture bottle, and the like. The bottom surface of the container may be transparent or opaque. When the bottom surface of the container is transparent, the observation, counting, etc. of cells can be made from the back side of the container. The container may have a solid or semisolid surface in its interior. As the solid surface, plates, containers, and the like of various materials like the above are given; as the semisolid surface, a gel, a soft polymer matrix, and the like are given. The culture substrate may be manufactured using any of the materials mentioned above, or a commercially available one may be used as the culture substrate. Preferred examples of the culture substrate include, but are not limited to, a substrate having an adherent surface suitable for the formation of a sheet-shaped cell culture. Specifically, a substrate having a hydrophilic surface is given, and examples include a substrate of which a surface is coated with a hydrophilic compound such as corona discharge-treated polystyrene, collagen gel, or a hydrophilic polymer, a substrate of which a surface is coated with any of extracellular matrices such as collagen, fibronectin, laminins, vitronectin, proteoglycans, and glycosaminoglycans, cell adhesion factors of the cadherin family, the selectin family, the integrin family, etc., and the like, and like substrates. Such substrates are commercially available (for example, Corning^{(R)} TC-Treated Culture Dish, Corning, etc.). The whole or part of the culture substrate may be transparent or opaque.

A surface of the culture substrate may be covered with a material of which physical properties change in response to a stimulus, for example temperature or light. Such materials may include, but are not limited to, known materials, such as temperature responsive materials each composed of a homopolymer or copolymer of a (meth)acrylamide compound, an N-alkyl-substituted (meth)acrylamide derivative (for example, N-ethylacrylamide, N-n-propylacrylamide, N-n-propylmethacrylamide, N-isopropylacrylamide, N-isopropylmethacrylamide, N-cyclopropylacrylamide, N-cyclopropylmethacrylamide, N-ethoxyethylacrylamide, N-ethoxyethylmethacrylamide, N-tetrahydrofurfurylacrylamide, or N-tetrahydrofurfurylmethacrylamide), an N,N-dialkylsubstituted (meth)acrylamide derivative (for example, N,N-dimethyl(meth)acrylamide, N,N-ethylmethylacrylamide, or N,N-diethylacrylamide), a (meth)acrylamide derivative having a cyclic group (for example, 1-(1-oxo-2-propenyl)pyrrolidine, 1-(1-oxo-2-propenyl)piperidine, 4-(1-oxo-2-propenyl)morpholine, 1-(1-oxo-2-methyl-2-propenyl)pyrrolidine, 1-(1-oxo-2-methyl-2-propenyl)piperidine, or 4-(1-oxo-2-methyl-2-propenyl)morpholine), or a vinyl ether derivative (for example, methyl vinyl ether); and light responsive materials each composed of a light absorbing polymer having azobenzene groups, a copolymer of a vinyl derivative of triphenyl methane leucohydrooxide and an acrylamide-based monomer, or an N-isopropylacrylamide gel containing spirobenzopyran (see, for example, JP H02-211865 A, and JP 2003-33177 A). Application of a predetermined stimulus to such a material can change the physical property of the material, for example, the hydrophilic property or hydrophobic property of the material, and to promote the detaching of the cell culture adhered on the material. Culture dishes coated with a temperature responsive material are commercially available (for example, UpCell^{(R)} CellSeed Inc.), and are usable in the production method of the present disclosure.

The culture substrate may have various shapes, but is preferably flat. The area of the culture substrate is not particularly limited, and may be, for example, approximately 1 cm² to approximately 200 cm², approximately 2 cm² to approximately 100 cm², approximately 3 cm² to approximately 50 cm², or the like. For example, a circular culture dish with a diameter of 10 cm is given as the culture substrate. In this case, the area is 56.7 cm².

The culture substrate may be coated (covered or coated) with a serum. A sheet-shaped cell culture with higher density can be formed by using a culture substrate coated with a serum. "Being coated with a serum" means a state where serum components are attached to a surface of a culture substrate. Such a state can be obtained by, for example, but not limited to, treating a culture substrate with a serum. The treatment with a serum includes bringing a serum into contact with the culture substrate and, as necessary, performing incubation for a prescribed period.

As the serum, xenogeneic serum and/or homologous serum may be used. In the case where the sheet-shaped cell culture is used for transplantation, the xenogeneic serum means a serum derived from an organism of a different species from the recipient. For example, in the case where the recipient is a human being, a serum derived from a cow or a horse, for example fetal calf serum (FBS or FCS), calf serum (CS), horse serum (HS), or the like, falls under the xenogeneic serum. The "homologous serum" means a serum derived from an organism of the same species as the recipient. For example, in the case where the recipient is a human being, human serum falls under the homologous serum. The homologous serum includes autologous serum (also referred to as autoserum), that is, a serum derived from the recipient, and homologous non-autologous serum derived from a homologous individual other than the recipient. In the present specification, sera other than autologous serum, that is, xenogeneic serum and homologous non-autologous serum may be collectively referred to as non-self serum.

The serum for coating the culture substrate may be obtained commercially, or may be prepared by a usual method from blood collected from a desired organism. Specific examples include a method in which collected blood is allowed to stand at room temperature for approximately 20 minutes to approximately 60 minutes and is coagulated, the resulting matter is centrifuged by approximately 1000 ×g to approximately 1200 ×g, and the supernatant is collected, and the like.

In the case where incubation is performed on the culture substrate, the serum may be used in a form of an undiluted solution, or may be used in a form of a diluted solution. The dilution may be performed with an arbitrary medium, examples of which include, but are not limited to, water, physiological saline solution, various buffer solutions (for example, PBS, HBSS, etc.), various liquid culture media (for example, DMEM, MEM, F12, DMEM/F12, DME, RPMI1640, MCDB (MCDB102, 104, 107, 120, 131, 153, 199, etc.), L15, SkBM, RITC80-7, etc.), and the like. The diluted concentration is not particularly limited as long as serum components can be attached on the culture substrate, and is, for example, approximately 0.5% to approximately 100% (v/v), preferably approximately 1% to approximately 60% (v/v), and more preferably approximately 5% to approximately 40% (v/v).

The incubation time is not particularly limited either as long as serum components can be attached on the culture substrate, and is, for example, approximately 1 hour to approximately 72 hours, preferably approximately 2 hours to approximately 48 hours, more preferably approximately 2 hours to approximately 24 hours, and still more preferably approximately 2 hours to approximately 12 hours. The incubation temperature is not particularly limited either as long as serum components can be attached on the culture substrate, and is, for example, approximately 0°C to approximately 60°C, preferably approximately 4°C to approximately 45°C, and more preferably room temperature to approximately 40°C.

The serum may be disposed of after incubation. As a technique for disposing of the serum, a common liquid disposal technique such as suction with a pipette or the like or decantation may be used. In a preferred embodiment of the present disclosure, after the serum is disposed of, the culture substrate may be cleaned with a serum-free cleaning liquid. The serum-free cleaning liquid is not particularly limited as long as it is a liquid medium that is free of serum and has no bad influence on the serum components attached to the culture substrate, and examples include, but are not limited to, water, physiological saline solution, various buffer solutions (for example, PBS, HBSS, etc.), various liquid culture media (for example, DMEM, MEM, F12, DMEM/F12, DME, RPMI1640, MCDB (MCDB102, 104, 107, 120, 131, 153, 199, etc.), L15, SkBM, RITC80-7, etc.), and the like. The cleaning technique may be a common technique for cleaning a culture substrate, and examples of techniques that can be used include, but are not limited to, a technique in which a serum-free cleaning liquid is added on a culture substrate, stirring is performed for a prescribed time (for example, approximately 5 seconds to approximately 60 seconds), and then the serum-free cleaning liquid is disposed of, and the like.

In the present disclosure, the culture substrate may be coated with a growth factor. Here, the "growth factor" means an arbitrary substance that accelerates the proliferation of cells as compared to the case where there is no growth factor, and examples include epidermal growth factor (EGF), vascular endothelium growth factor (VEGF), fibroblast cells growth factor (FGF), and the like. The technique for coating the culture substrate with a growth factor, the disposal technique, and the cleaning technique are basically the same as those for serum except that the diluted concentration at the time of incubation is, for example, approximately 0.0001 µg/mL to approximately 1 µg/mL, preferably approximately 0.0005 µg/mL to approximately 0.05 µg/mL, and more preferably approximately 0.001 µg/mL to approximately 0.01 µg/mL.

In the present disclosure, the culture substrate may be coated with a steroid. Here, the "steroid" means, among compounds having a steroid nucleus, one that can have bad influence such as adrenal cortical insufficiency or Cushing's syndrome on a living body. Examples of such a compound include, but are not limited to, cortisol, prednisolone, triamcinolone, dexamethasone, betamethasone, and the like. The technique for coating the culture substrate with a steroid, the disposal technique, and the cleaning technique are basically the same as those for serum except that the diluted concentration at the time of incubation is, on a dexamethasone basis, for example, approximately 0.1 µg/mL to approximately 100 µg/mL, preferably approximately 0.4 µg/mL to approximately 40 µg/mL, and more preferably approximately 1 µg/mL to approximately 10 µg/mL.

The culture substrate may be coated with any one of a serum, a growth factor, and a steroid, or may be coated with an arbitrary combination of these, that is, a combination of a serum and a growth factor, a combination of a serum and a steroid, a combination of a serum, a growth factor, and a steroid, or a combination of a growth factor and a steroid. In the case where coating is performed with a plurality of components, these components may be mixed and simultaneously used for coating, or may be used for coating in separate steps.

The culture substrate may be seeded with cells immediately after coated with a serum or the like, or may be preserved after coated and then be seeded with cells. The coated substrate can be preserved for a long period by being kept at, for example, approximately not more than 4°C, preferably approximately not more than -20°C, and more preferably approximately not more than -80°C.

The seeding of cells on the culture substrate may be performed by a known arbitrary technique and known arbitrary conditions. The seeding of cells on the culture substrate may be performed also by, for example, injecting, into the culture substrate (a culture container), a cell suspension in which cells are suspended in a culture solution. Tools suitable for the manipulation of cell suspension injection, such as a syringe or a pipette, may be used for the injection of a cell suspension.

In the present disclosure, "detaching" the sheet-shaped cell culture means stripping (removing) the sheet-shaped cell culture from the culture substrate, or the sheet-shaped cell culture coming off. That is, not only actively stripping the sheet-shaped cell culture from the culture substrate but also, for example, the intercellular binding force of the sheet-shaped cell culture exceeding the binding force between the sheet-shaped cell culture and the culture surface to cause spontaneous coming-off is included in "detaching".

In the present disclosure, "soaking" means a state where the sheet-shaped cell culture is soaked in a low nutrient isotonic solution. That is, any state where the sheet-shaped cell culture is covered with liquid is an "soaked" state, and even a state where part of the sheet is exposed to the outside of liquid in a still-standing state falls under an "soaked" state if the part enters a state of being covered with liquid by, for example, shaking.

In the present disclosure, the sheet-shaped cell culture's "shrinkage" means that, when the sheet-shaped cell culture adhered to the culture substrate is detached from the substrate, the area of the sheet-shaped cell culture becomes smaller after the detaching.

In the present disclosure, the sheet-shaped cell culture's "shrinking" means that an outer edge portion of the sheet-shaped cell culture becomes round or twisted. Such shrinkage is caused by the action of intercellular adhesion force, and is different from wrinkles or folds that physically occur due to detaching working or transfer working. If the sheet-shaped cell culture is left in a shrunken state, the sheet-shaped cell culture experiences adhesion or sticking from the part that has become round or twisted.

In the present disclosure, "extending" means extending and eliminating wrinkles, folds, shrinkage, twists, etc. that have occurred in the sheet-shaped cell culture.

In the present disclosure, the term "isotonic" means a state in which two kinds of liquids are equivalent in osmotic pressure, however, the term "isotonic" as used herein means "physiologically isotonic" unless specified otherwise, and means to have an osmotic pressure equivalent to that of a physiological liquid, such as an intracellular fluid and blood. Thus, in the present disclosure, the term "isotonic solution" has the same meaning as the term "physiologically isotonic solution" unless specified otherwise, and means a liquid having an osmotic pressure equivalent to that of a physiological liquid, such as an intracellular fluid and blood. Examples of the isotonic solution include, but not limited to, Hanks' balanced salt solution, saline, phosphate-buffered physiological saline, ringer solution, basal medium and the like.

In the present disclosure, the term "low nutrient" or "low nutrient state" means conditions under which the cell count of a cell population placed under the conditions remains with neither proliferation nor extinction for a predetermined period, in other words, conditions under which the cell count remains substantially unchanged for a predetermined period. The expression "the cell count remains substantially unchanged" means that there is no substantial difference between a cell count A at a time point and a cell count B at another time point after a lapse of a predetermined period from the former time point. Described specifically, "the cell count remains substantially unchanged" means several cases where the cell count A is approximately 70%, approximately 80%, approximately 90%, approximately 95%, approximately 100%, approximately 105%, approximately 110%, approximately 120%, approximately 130% or the like of the cell count B, for example. These two time points may be arbitrarily selected. However, when culturing is conducted under normal culturing conditions, it is preferred to leave an interval to such an extent that proliferation of cells can be confirmed. Such an interval may be, for example, three days, four days, five days, six days, seven days or the like.

The low nutrient state may be attained under various conditions. Any of those skilled in the art can appropriately create a low nutrient state in accordance with desired cells. The requirement or requirements of a low nutrient state may typically include that energy required for the life support of cells can be supplied, that elements required for cell division is lacked, and/or the like, for example. Specific examples may include an environment where energy, such as a sugar, required for metabolism can be supplied, an environment where an essential amino acid required for cell division cannot be supplied, and/or the like.

In an embodiment, "low nutrient" means a low sugar. The term "low sugar" means a state where a saccharide is contained but the proportion of saccharide is relatively low. Accordingly, a saccharide-free state is not encompassed in the meaning of the low sugar. In a specific example of such an embodiment, the low sugar is a low glucose and the low glucose is not glucose free, for example. Typical examples of the low sugar include a composition in which saccharides are contained less than 1000 mg/L, and the like, for example. Examples of another solution of low sugar conditions include a condition where the content of saccharoids has been lowered to less than 1% in comparison with the conditions for saccharoids in general culture solutions that contain no additional saccharoid. In example, the amount of saccharides contained in the low sugar state solution may be specifically less than 1000 mg/L, preferably less than 500 mg/L, more preferably less than 200 mg/L, still more preferably less than 100 mg/mL.

In another embodiment, "low nutrient" means an amino acid-free state, that is, containing no amino acid. As a specific example of the low nutrient in such an embodiment, no essential amino acid is contained.

In the present disclosure, the term "low nutrient isotonic solution" means an isotonic solution which is low in nutrients. The low nutrient isotonic solution can be an isotonic solution containing a predetermined amount of carbohydrates, and/or an isotonic solution incapable of supplying essential amino acids, for example. The low nutrient isotonic solution is specifically an isotonic solution containing a predetermined amount of saccharides but containing no amino acid (especially, essential amino acid), an isotonic solution containing a predetermined amount of saccharides and a synthesis inhibitor for essential amino acids and the like, for example. The carbohydrate that can be contained in the low nutrient isotonic solution is typically saccharide. Examples of the saccharide may include but are not limited to, glucose, sucrose, maltose, fructose, galactose or the like, and can be appropriately selected depending on the desired type of cells or the like. The predetermined content of a saccharide may vary depending on the type of the contained saccharide or the required retention period (soaking time) of cells. In the case of glucose, for example, from the viewpoint of the amount required for the life support of cells, the saccharide content may, for example, be approximately not less than 100 mg/L, approximately not less than 500 mg/L, approximately not less than 1000 mg/L, approximately not less than 1500 mg/L, approximately not less than 2000 mg/L, or the like. From the viewpoint of the maintenance of isotonicity, the saccharide content may, for example, be approximately not more than 50000 mg/L, approximately not more than 40000 mg/L, approximately not more than 30000 mg/L, approximately not more than 25000 mg/L, approximately not more than 20000 mg/L, approximately not more than 15000 mg/L, approximately not more than 10000 mg/L, approximately not more than 5000 mg/L, approximately not more than 4500 mg/L, or the like. Thus, the example of the range of glucose content may include any desired combinations of these upper limits and these lower limits, and may include, for example, but are not limited to, approximately 100 mg/L to 500000 mg/L, approximately 500 mg/L to 25000 mg/L, approximately 500 mg/L to 4500 mg/L, approximately 1500 mg/L to 4500 mg/L, approximately 500 mg/L to 1500 mg/L. It is possible for those skilled in the art to calculate the predetermined content according to the type of the contained saccharide.

Examples of the low nutrient isotonic solution may include those each prepared by adding a predetermined amount of carbohydrate to an isotonic solution known to have a composition without any carbohydrate nor any amino acid, such as saline, phosphate-buffered physiological saline, and ringer solution, in addition to isotonic solutions each known to have a composition containing a predetermined amount of saccharide but containing no amino acid, such as Hanks' balanced salt solution, Earl's balanced salt solution, and glucose isotonic solutions. As the predetermined amount of carbohydrate, 1000 mg/L to 4500 mg/L of glucoses is generally contained, for example, in the case of Hanks' balanced salt solution or Earl's balanced salt solution, or approximately 50000 mg/L of glucose is generally contained, for example, in the case of a glucose isotonic solution. In an embodiment, the term "low nutrient isotonic solution" does not include stock solutions existing known as cold storage solutions for organs, such as UW solution, Euro-Collins solution, and Hypothermosol (registered trademark).

The low nutrient isotonic solution of the present disclosure may contain lactic acid. Thus, in an embodiment of the present disclosure, the low nutrient isotonic solution contains no lactic acid. In addition, the low nutrient isotonic solution of the present disclosure may contain pyruvic acid. Thus, in an embodiment of the present disclosure, the low nutrient isotonic solution contains no pyruvic acid. In addition, the low nutrient isotonic solution of the present disclosure may contain ascorbic acid. Thus, in an embodiment of the present disclosure, the low nutrient isotonic solution contains ascorbic acid. In addition, the low nutrient isotonic solution of the present disclosure may contain fatty acid. Thus, in an embodiment of the present disclosure, the low nutrient isotonic solution contains no fatty acid. In addition, the low nutrient isotonic solution of the present disclosure may contain calcium. Thus, in an embodiment of the present disclosure, the low nutrient isotonic solution contains calcium, for example, at a concentration preferably of approximately 1 mM to 2 mM, more preferably of approximately 1.2 mM to 1.8 mM. In addition, the low nutrient isotonic solution of the present disclosure may contain cholesterol. Thus, in an embodiment of the present disclosure, the low nutrient isotonic solution contains no cholesterol. In a preferred embodiment, the low nutrient isotonic solution contains none of lactic acid, pyruvic acid, fatty acid, and cholesterol, but contains ascorbic acid, and contains calcium, for example, at a concentration of approximately 1 mM to 2 mM, preferably of approximately 1.2 mM to 1.8 mM.

In the present disclosure, the term "carbohydrate" is a generic term for organic compounds formed of a monosaccharide as a constituent unit, and encompasses saccharide derivatives and the like in addition to monosaccharides, oligosaccharides, and polysaccharides. In this description, the terms "saccharide" and "sugar" mean among carbohydrates, those which are metabolized by cells and converted to energy as well as their components typically represent monosaccharides and also include those which are degraded in the system and metabolized by cells.

In the present disclosure, "normal temperature" is 15°C to 25°C.

### <1> Shrinkage suppression method of the present disclosure

An aspect of the present disclosure provides a method for suppressing the shrinkage of a sheet-shaped cell culture formed on a culture substrate when detaching the sheet-shaped cell culture (hereinafter, written as a detaching method of the present disclosure).

The method for detaching a sheet-shaped cell culture of the present disclosure includes the following steps:
(a) Soaking, in a low nutrient isotonic solution, the upper surface of a sheet-shaped cell culture of which at least part is adhered to a culture substrate.

In step (a), the soaking in a low nutrient isotonic solution of the upper surface of a sheet-shaped cell culture of which at least part is adhered to a culture substrate is typically achieved by putting the low nutrient isotonic solution into the culture container. The sheet-shaped cell culture to be soaked may be in a state where at least part of it is adhered to the culture substrate, but is preferably in a state where the whole area is adhered to the culture substrate. In an embodiment, the soaking of a sheet-shaped cell culture in a low nutrient isotonic solution is achieved by removing a culture medium that has been used for the formation of the sheet-shaped cell culture and putting in the low nutrient isotonic solution, that is, substituting the culture medium with the low nutrient isotonic solution.

An embodiment of the detaching method of the present disclosure arbitrarily includes step (b) below subsequently to step (a) above:
(b) Detaching the sheet-shaped cell culture in the low nutrient isotonic solution.

In step (b), the sheet-shaped cell culture adhered to the culture substrate and soaked in the low nutrient isotonic solution may be spontaneously detached by still standing in a state of soaking in the low nutrient isotonic solution, or may be actively detached from the culture substrate in the low nutrient isotonic solution. In a preferred embodiment, the sheet-shaped cell culture is actively detached. The method of active detaching is not particularly limited unless it is one that injures the sheet-shaped cell culture, and examples include a method of shaking the low nutrient isotonic solution together with the substrate, a method of jetting the low nutrient isotonic solution (by, for example, pipetting or the like) to an adhesion edge of the sheet-shaped cell culture adhered to the substrate and thereby detaching the sheet-shaped cell culture, and the like.

The detaching method of the present disclosure can avoid a situation where, when detaching the sheet-shaped cell culture, an already detached end portion of the sheet-shaped cell culture detached from the substrate twists or shrinks while detaching is being performed and is stuck or conglutinated as it is. This is presumed to be because the physiological working of cells contained in the sheet-shaped cell culture is reduced by soaking in the low nutrient isotonic solution and consequently intercellular adhesion force is weakened.

In an embodiment of the detaching method of the present disclosure, as described above the soaking is performed by removing a culture medium that has been used for the formation of the sheet-shaped cell culture and substituting the culture medium with a low nutrient isotonic solution. In such an embodiment, production step-derived impurities remaining in the sheet-shaped cell culture can be removed by performing the soaking. In the present disclosure, "production step-derived impurities" typically include those enumerated below derived from steps in the production stage of the sheet-shaped cell culture. That is, they are those derived from the cell substrate (for example, host cell-derived proteins, host cell-derived DNAs, etc.), those derived from the cell culture solution (for example, inducers, antibiotics, culture medium components, xenogeneic serum, etc.), those derived from the steps of extraction, separation, processing, and purification of the object substance, which are steps after cell culture, and the like (for example, see "Iyaku Shin Hatsu Dai 571 Go" (Notification No. 571 issued by the Director of the Evaluation and Licensing Division, the Pharmaceutical Bureau, the Ministry of Health, Labour and Welfare of Japan)).

The detaching method of the present disclosure may arbitrarily further include step (c) below:
(c) A step of extending the sheet-shaped cell culture.

Step (c) is a step of extending a sheet-shaped cell culture twisted or bent. The extending step (c) may be performed after the detaching step (b). The extending step (c) may be performed in the low nutrient isotonic solution; alternatively, it is possible to remove the low nutrient isotonic solution, then perform the extending step (c), and then add the low nutrient isotonic solution again. In the case where the extending step (c) is performed after the low nutrient isotonic solution is removed, step (c) may be performed by, for example, a technique such as dropping the low nutrient isotonic solution onto an overlapping part of the sheet-shaped cell culture and, as necessary, performing shaking. In the case where the extending step (c) is performed in the low nutrient isotonic solution, step (c) may be performed by a technique such as shaking the whole container containing the sheet-shaped cell culture soaked in the low nutrient isotonic solution. From the viewpoints of simplicity, etc., the extending step is preferably performed, in the low nutrient isotonic solution, by shaking the container containing the low nutrient isotonic solution in which the sheet-shaped cell culture is soaked.

The extending method of the present disclosure is not limited as long as the sheet-shaped cell culture can be extended; for example, rotational shaking is preferable from the viewpoint of the ability of extending without unevenness. In the rotational shaking, it is presumed that, by rotating, in the container, the liquid in which the sheet-shaped cell culture is soaked, centrifugal force is generated in the liquid, and the sheet-shaped cell culture is pulled to the outside by such centrifugal force and the frictional force between the sheet-shaped cell culture and the liquid, and can consequently be extended. Thus, in the rotational shaking, it is preferable that the liquid in the container rotate with the middle of the container as the center.

As the method for rotating liquid in shaking, any method known in the relevant technical field may be used to the extent that the sheet-shaped cell culture existing in liquid is prevented from breakage. Specific examples include, but are not limited to, a method of rotating or rotationally shaking the container containing liquid, a method of rotating a stirring bar or the like in liquid, and the like. From the viewpoints of little risk of directly damaging the sheet-shaped cell culture, little risk of contamination, etc., a method of rotating or rotationally shaking the container without direct contact with the liquid and thereby rotating the internal liquid is preferable. In view of force transfer efficiency, it is most preferable to rotationally shake the container. In the present invention, "rotating" a container refers to rotating the container itself on its axis, and rotationally shaking a container refers to circularly moving the container and shaking the container.

In the rotational shaking of the present disclosure, centrifugal force generated when the liquid in which the sheet-shaped cell culture is soaked is rotated and the frictional force between the liquid and the sheet-shaped cell culture are used as force for extending; therefore, there is no case where force becomes extremely strong or a load is unevenly applied to the sheet-shaped cell culture. Thus, the rotational shaking of the present disclosure can be suitably used particularly in the case where the sheet-shaped cell culture is a fragile sheet-shaped cell culture.

The shaking force and the shaking time are determined by, for example, shaking and the amplitude; if the shaking force is too large or if the shaking time is too long, the risk of breakage the sheet-shaped cell culture is increased; however, the shaking force and the shaking time may vary with the shape and the kind of the container used, the fragility of the sheet-shaped cell culture, the amount and the kind of the liquid, etc.; a person skilled in the art can determine appropriate numerical values, as appropriate. In the case where the sheet-shaped cell culture is skeletal myoblasts, about approximately 5 rpm to 300 rpm are appropriate. For example, to eliminate bends or wrinkles in very small parts that have occurred in margins of the sheet-shaped cell culture at the time of detaching, extending at approximately 30 rpm to 50 rpm is preferable; in the case where twists or shrinkage has occurred, extending at approximately 30 rpm to 90 rpm, at which the sheet-shaped cell culture does not break while the liquid in the container rotates sufficiently, is preferable; the range whereby adhesion or sticking can be extended is preferably approximately 90 rpm to 130 rpm, which is a little strong. As the shaking time, in the case of 30 rpm to 50 rpm as an example, approximately 1 to 15 minutes are preferable; in the case of 30 rpm to 90 rpm, approximately 1 to 10 minutes are preferable; in the case of 90 rpm to 130 rpm, approximately 1 to 8 minutes are preferable.

The extending step (c) may be performed at normal temperature, or may be performed under cold storage conditions, for example, at 0 to 10°C, 2 to 8°C, or the like. In the case where a low nutrient isotonic solution is used for the extending, also such a low nutrient isotonic solution may be at normal temperature, or may be under cold storage conditions, for example at 0 to 10°C, or 2 to 8°C. From the viewpoint of reducing the physiological working of cells, 0 to 10°C are preferable.

Any of the culture substrates described above may be used as the culture substrate used for the detaching method of the present disclosure. In a preferred embodiment, the culture substrate is covered with a serum. In another preferred embodiment, the culture substrate is covered with a temperature-responsive material. In a more preferred embodiment, the culture substrate is covered with a temperature-responsive material and a serum.

Any of the cells described above may be used as the cell used for the sheet-shaped cell culture to be detached by the detaching method of the present disclosure. In a preferred embodiment, the sheet-shaped cell culture contains myoblast cells. In another preferred embodiment, the sheet-shaped cell culture contains myocardiac cells (cardimyocytes). In a more preferred embodiment, the myocardiac cell contained in the sheet-shaped cell culture is a myocardiac cell derived from an iPS cell.

Any of the low nutrient isotonic solutions described above may be used as the low nutrient isotonic solution used for the detaching method of the present disclosure. In a preferred embodiment, the low nutrient isotonic solution is Hanks' balanced salt solution (HBSS), particularly preferably HBSS(+).

From the viewpoint of reducing the physiological activities of cells contained in the sheet-shaped cell culture, the low nutrient isotonic solution is preferably at not more than room temperature. Further, from the viewpoint of limiting damage to the cell as small as possible, the low nutrient isotonic solution is preferably not less than 0°C. In a preferred embodiment, the low nutrient isotonic solution is at 0 to 10°C, more preferably 2 to 8°C.

An aspect of the present disclosure provides a method for suppressing the shrinkage of a sheet-shaped cell culture detached from a culture substrate by perfusing the sheet-shaped cell culture (hereinafter, written as a perfusion method of the present disclosure).

The method for perfusing a sheet-shaped cell culture of the present disclosure includes the following steps:
(A) Perfusing a detached sheet-shaped cell culture with a low nutrient isotonic solution.

In the present disclosure, "perfusion" refers to passing a liquid to a surface or the interior of a tissue, a graft, or the like to remove serum, culture medium components, etc (rinse a surface or the interior of a tissue, a graft, or the like with a flow of liquid).

Step (A) is performed at least once, and may be repeated multiple times, for example may be repeated once, twice, three times, four times, five times, or six times. In a preferred embodiment, perfusion is performed three times.

The perfusion method of the present disclosure is a method in which a sheet-shaped cell culture after detaching is perfused with a low nutrient isotonic solution and thereby the shrinkage of the sheet-shaped cell culture detached from the substrate is suppressed. Usually, after a sheet-shaped cell culture is detached from a culture substrate, the sheet-shaped cell culture in an isolated state has been tensed due to the intercellular adhesion force between constituent cells and therefore has experienced shrinkage, and is likely to develop twists or shrinkage during the course. However, in the method of the present disclosure, the physiological activites of cells is reduced by performing perfusion with a low nutrient isotonic solution, and thereby intercellular adhesion force is weakened; thus, twists or shrinkage can be suppressed effectively. Further, by performing perfusion in a low nutrient isotonic solution, even if an overlap occurs in the sheet-shaped cell culture due to a twist, a bend, or the like, the place can be saved from experiencing adhesion or sticking; thus, a high-quality sheet-shaped cell culture can be provided.

The perfusion of the sheet-shaped cell culture of the present disclosure is performed by bringing a low nutrient isotonic solution into contact with a surface of the detached sheet-shaped cell culture. Such perfusion may be performed by continuing to pass a low nutrient isotonic solution to a surface of the detached sheet-shaped cell culture, or may be performed by soaking the detached sheet-shaped cell culture in a low nutrient isotonic solution. In a preferred embodiment, perfusion is performed on both surfaces, for example the upper surface and the lower surface, of the detached sheet-shaped cell culture. Further, perfusion may be performed alternately on the upper surface and the lower surface of the detached sheet-shaped cell culture. In another preferred embodiment, perfusion is performed by soaking the detached sheet-shaped cell culture in a low nutrient isotonic solution. The soaking of the sheet-shaped cell culture in a low nutrient isotonic solution may be implemented by introducing the detached sheet-shaped cell culture into the low nutrient isotonic solution, or may be implemented by putting the low nutrient isotonic solution into a container containing the detached sheet-shaped cell culture. From the viewpoint of reducing the risk of twists, breakage, etc. caused by directly manipulating the sheet-shaped cell culture, perfusion is preferably implemented by putting a low nutrient isotonic solution into a container containing the detached sheet-shaped cell culture and soaking the sheet-shaped cell culture.

In the case where the sheet-shaped cell culture is soaked in a low nutrient isotonic solution by putting the low nutrient isotonic solution into a container containing the detached sheet-shaped cell culture, it is preferable that the low nutrient isotonic solution be put into a culture container in which the sheet-shaped cell culture has been formed. That is, a sheet-shaped cell culture is formed on a culture substrate in a culture container, then the sheet-shaped cell culture is detached, and then a low nutrient isotonic solution is put into the culture container without moving the detached sheet-shaped cell culture. In the case where the detaching of the sheet-shaped cell culture from the culture substrate has been implemented in a liquid, it is preferable that a low nutrient isotonic solution be put in after the liquid used for detaching is removed.

An embodiment of the perfusion method of the present disclosure arbitrarily includes step (B) below subsequently to (A) mentioned above:
(B) Removing the low nutrient isotonic solution, putting in a new low nutrient isotonic solution, and soaking the sheet-shaped cell culture again.

Soaking in a low nutrient isotonic solution may be performed multiple times. In the case where such soaking is performed multiple times, it may be performed by, for example, introducing the sheet-shaped cell culture successively into a plurality of containers each containing a low nutrient isotonic solution, or by repeating multiple times a procedure of putting a low nutrient isotonic solution into a container containing the sheet-shaped cell culture and performing soaking, then removing the low nutrient isotonic solution, and putting in a new low nutrient isotonic solution.

Step (B) is performed at least once, and may be repeated multiple times, for example may be repeated once, twice, three times, four times, five times, or six times. In a preferred embodiment, re-soaking is performed three times.

In general, after a sheet-shaped cell culture is formed on a culture substrate, shrinkage force occurs in the sheet-shaped cell culture due to intercellular adhesion force acting strongly. When such shrinkage force exceeds the adhesion force between the culture substrate and the sheet-shaped cell culture, the sheet-shaped cell culture detaches spontaneously. The present inventors have found that such shrinkage force strongly acts for approximately 12 hours from the start of sheet formation. Therefore, if the sheet-shaped cell culture is detached from the culture substrate before 12 hours have elapsed from the start of sheet formation, strong shrinkage force acts, and the area of the sheet-shaped cell culture becomes smaller (that is, the sheet-shaped cell culture shrinks). Further, even if the sheet-shaped cell culture is detached when or after 12 hours have elapsed from the start of sheet formation, the sheet-shaped cell culture shrinks to some degree likewise, although the action of shrinkage force is weaker and accordingly the change in the area of the sheet-shaped cell culture is not larger than in the case of before 12 hours have elapsed from the start of sheet formation. Due to such shrinkage, the sheet-shaped cell culture develops twists or shrinkage. By the perfusion method of the present disclosure, twists or shrinkage occurring due to such shrinkage force, and consequent adhesion or the like can be suppressed. Thus, in a preferred embodiment, the perfusion method of the present disclosure is implemented before 12 hours have elapsed from the start of sheet formation. In another preferred embodiment, the perfusion method of the present disclosure is performed during the period from immediately after the sheet-shaped cell culture is detached from the culture substrate to when 6 hours have elapsed from the detaching.

An embodiment of the perfusion method of the present disclosure may arbitrarily further include step (C) below:
(C) Extending the sheet-shaped cell culture.

Step (C) means a step of extending a sheet-shaped cell culture that is twisted or bent during the step of detaching or removal of liquid. The extending step (C) may be performed before the perfusion step (A) or the re-perfusion step (B), after the perfusion step (A) or the re-perfusion step (B), or between the perfusion step (A) and the re-perfusion step (B), or between steps (B) in the case where there are a plurality of times of re-perfusion steps (B). The extending step (C) may be performed in the low nutrient isotonic solution; alternatively, it is possible to remove the low nutrient isotonic solution, then perform the extending step (C), and then add the low nutrient isotonic solution again. In the case where the extending step (C) is performed after the low nutrient isotonic solution is removed, step (C) may be performed by, for example, a technique such as dropping the low nutrient isotonic solution onto an overlapping part of the sheet-shaped cell culture and, as necessary, performing shaking. In the case where the extending step (C) is performed in the low nutrient isotonic solution, step (C) may be performed by a technique such as shaking the whole container containing the sheet-shaped cell culture soaked in the low nutrient isotonic solution. From the viewpoints of simplicity, etc., the extending step (C) is preferably performed by shaking the container containing the low nutrient isotonic solution in which the sheet-shaped cell culture is soaked.

The extending step (C) may be performed at normal temperature, or may be performed under cold storage conditions, for example at 0 to 10°C, 2 to 8°C, or the like. In the case where a low nutrient isotonic solution is used for the extending, also such a low nutrient isotonic solution may be at normal temperature, or may be under cold storage conditions, for example at 0 to 10°C, or 2°C to 8°C.

An embodiment of the perfusion method of the present disclosure may arbitrarily further include step (D) below:
(D) Soaking the sheet-shaped cell culture in a low nutrient isotonic solution and preserving the sheet-shaped cell culture as it is.

Step (D) may be performed at any time after step (A), but is preferably performed at the very end after the other steps have ended.

Any of the cells described above may be used as the cell used for the sheet-shaped cell culture to be provided for the perfusion method of the present disclosure. In a preferred embodiment, the sheet-shaped cell culture contains myoblast cells. In another preferred embodiment, the sheet-shaped cell culture contains myocardiac cells. In a more preferred embodiment, the myocardiac cell contained in the sheet-shaped cell culture is a myocardiac cell derived from an iPS cell.

Any of the low nutrient isotonic solutions described above may be used as the low nutrient isotonic solution used for the perfusion method of the present disclosure. In a preferred embodiment, the low nutrient isotonic solution is preferably a buffer solution, more preferably Hanks' balanced salt solution (HBSS), and particularly preferably HBSS(+).

From the viewpoint of reducing the physiological activities of cells contained in the sheet-shaped cell culture, the low nutrient isotonic solution is preferably at not more than room temperature. Further, from the viewpoint of limiting damage to the cell as small as possible, the low nutrient isotonic solution is preferably not less than 0°C. In a preferred embodiment, the low nutrient isotonic solution is at 0 to 10°C, more preferably 2 to 8°C.

An aspect of the present disclosure may include only steps (A), (B), and (D).

An aspect of the present disclosure provides a method for suppressing shrinkage when preserving a sheet-shaped cell culture detached from a culture substrate (hereinafter, written as a preservation method of the present disclosure).

The method for preserving a sheet-shaped cell culture of the present disclosure includes the following steps:
(i) soaking a detached sheet-shaped cell culture in a low nutrient isotonic solution. The soaking step (i) may be performed at normal temperature, or may be performed under cold storage conditions, for example at 0 to 10°C, 2 to 8°C, or the like. The temperature of the low nutrient isotonic solution used for soaking is not particularly limited as long as there is no damage to the cell contained in the sheet-shaped cell culture, and may be normal temperature, or may be under cold storage conditions, for example 0 to 10°C, 2 to 8°C, or the like. In a preferred embodiment, at the time of the start of soaking, the low nutrient isotonic solution is under cold storage conditions, for example at 0 to 10°C, or 2 to 8°C. In another preferred embodiment, at the time of the start of soaking, the low nutrient isotonic solution is at normal temperature. The temperature of the low nutrient isotonic solution may vary during preservation.

In the case where the sheet-shaped cell culture is used for regenerative medicine or the like, the sheet-shaped cell culture is preferably used promptly after it is detached from the culture substrate because the quality of the sheet-shaped cell culture decreases with the lapse of time, but in practice the sheet-shaped cell culture needs to be preserved for a certain time before use. The preservation method of the present disclosure is a method in which a sheet-shaped cell culture after detaching is soaked in a low nutrient isotonic solution and thereby the sheet-shaped cell culture is allowed to be preserved for a certain time without reducing the quality of the sheet-shaped cell culture. As factors of quality reduction, reduction in the activity of cells contained in the sheet-shaped cell culture, change in physical characteristics of the sheet-shaped cell culture, etc. are given. For example, after a sheet-shaped cell culture is detached from a substrate, the sheet-shaped cell culture has been tensed due to the intercellular adhesion force between constituent cells, and is likely to experience shrinkage. In the preservation method of the present disclosure, the physiological activities of cells is moderately reduced by preserving the sheet-shaped cell culture while keeping it soaked in a low nutrient isotonic solution, and thereby intercellular adhesion force is weakened; thus, shrinkage can be lessened. Further, by performing soaking in a low nutrient isotonic solution, even if an overlap occurs in the sheet-shaped cell culture due to a twist, a bend, or the like, the place can be saved from experiencing adhesion or sticking; thus, a high-quality sheet-shaped cell culture can be provided.

In the present disclosure, "soaking" means a state where the sheet-shaped cell culture is soaked (immersed) in a low nutrient isotonic solution. That is, any state where the sheet-shaped cell culture is covered with liquid is an "soaked" state, and even a state where part of the sheet is exposed to the outside of liquid in a still-standing state falls under an "soaked" state if the part is a state of being covered with liquid by, for example, shaking.

The soaking of the sheet-shaped cell culture may be implemented by introducing the detached sheet-shaped cell culture into a low nutrient isotonic solution contained in a preservation container, or may be implemented by putting a low nutrient isotonic solution into a preservation container containing the detached sheet-shaped cell culture; however, from the viewpoint of reducing the risk of twists, breakage, etc. caused by directly manipulating the sheet-shaped cell culture, soaking is preferably implemented by putting a low nutrient isotonic solution into a preservation container containing the detached sheet-shaped cell culture.

The preservation container may be a container prepared for preservation separately from the culture container, or the culture container may be used for preservation as it is. From the viewpoint of reducing the risk of twists, breakage, etc. caused by directly manipulating the sheet-shaped cell culture, it is preferable that the culture container be used for preservation as it is. That is, a sheet-shaped cell culture is formed on a culture substrate in a culture container, then the sheet-shaped cell culture is detached and cleaned, and then a low nutrient isotonic solution is put into the culture container without moving the detached sheet-shaped cell culture.

An embodiment of the preservation method of the present disclosure may arbitrarily further include step (ii) below:
(ii) Extending the sheet-shaped cell culture.

Step (ii) means a step of extending a sheet-shaped cell culture that is twisted or bent during the step of detaching or cleaning. The extending step (ii) may be performed before the soaking step (i), after the soaking step (i), or during the soaking. The extending step (ii) may be performed in the low nutrient isotonic solution; alternatively, it is possible to remove the low nutrient isotonic solution, then perform the extending step (ii), and then add the low nutrient isotonic solution again. In the case where the extending step is performed after the low nutrient isotonic solution is removed, step may be performed by, for example, a technique such as dropping the low nutrient isotonic solution onto an overlapping part of the sheet-shaped cell culture and, as necessary, performing shaking. In the case where the extending step (ii) is performed in the low nutrient isotonic solution, step (ii) may be performed by a technique such as shaking the whole container containing the sheet-shaped cell culture soaked in the low nutrient isotonic solution. From the viewpoints of simplicity, etc., the extending step (ii) is preferably performed by shaking the container containing the low nutrient isotonic solution in which the sheet-shaped cell culture is soaked.

The extending step (ii) may be performed at normal temperature, or may be performed under cold storage conditions, for example at 0 to 10°C, 2 to 8°C, or the like. In the case where a low nutrient isotonic solution is used for the extending, also such a low nutrient isotonic solution may be at normal temperature, or may be under cold storage conditions, for example at 0 to 10°C, or 2 to 8°C. Preferably at normal temperature.

Any of the cells described above may be used as the cell used for the sheet-shaped cell culture to be preserved by the preservation method of the present disclosure. In a preferred embodiment, the sheet-shaped cell culture contains myoblast cells. In another preferred embodiment, the sheet-shaped cell culture contains myocardiac cells. In a more preferred embodiment, the myocardiac cell contained in the sheet-shaped cell culture is a myocardiac cell derived from an iPS cell.

Any of the low nutrient isotonic solutions described above may be used as the low nutrient isotonic solution used for the preservation method of the present disclosure. In a preferred embodiment, the low nutrient isotonic solution is Hanks' balanced salt solution (HBSS), particularly preferably HBSS(+).

In the preservation method of the present disclosure, the preservation temperature is not particularly limited as long as there is no damage to the cell contained in the sheet-shaped cell culture; however, from the viewpoint of simplicity during the use of the sheet-shaped cell culture after preservation, the preservation temperature is preferably not under freezing conditions (temperatures at which the low nutrient isotonic solution freezes). From the viewpoint of simplicity of the preservation manipulation itself, the preservation temperature is preferably normal temperature.

### <2> Production method of the present disclosure

An aspect of the present disclosure provides a method for producing a sheet-shaped cell culture including all of the detaching method, the perfusion method, and the preservation method of the present disclosure mentioned above.

A method for producing a sheet-shaped cell culture of the present disclosure includes steps (I) to (V) below:
(I) Seeding cells for constituting a sheet-shaped cell culture on a culture substrate at a density at which a sheet-shaped cell culture can be formed with practically no proliferation;
(II) Making the cells seeded in (I) into a sheet in a cell culture solution and forming a sheet-shaped cell culture;
(III) Soaking the sheet-shaped cell culture obtained in (II) in a low nutrient isotonic solution and detaching the sheet-shaped cell culture
(IV) Removing the low nutrient isotonic solution in which the detached sheet-shaped cell culture is soaked and putting in a new low nutrient isotonic solution
(V) Preserving the sheet-shaped cell culture soaked in a low nutrient isotonic solution

As the culture substrate, the cell, and the low nutrient isotonic solution of the production method of the present disclosure, those described above may be used.

In the step (I), the seeding of cells on the culture substrate may be performed by a known arbitrary technique and known arbitrary conditions. The seeding of cells on the culture substrate may be performed also by, for example, injecting, into the culture substrate (a culture container), a cell suspension in which cells are suspended in a culture solution. Tools suitable for the manipulation of cell suspension injection, such as a syringe or a pipette, may be used for the injection of a cell suspension.

In an embodiment, the cell population to be seeded contains one or more types of cells selected from the cells constituting the above-described sheet-shaped cell culture, preferably two or more types of cells selected from the cells forming the above-described sheet-shaped cell culture.

The content percentage (purity) of cells of the most common type among the two or more types of cells forming the sheet-shaped cell culture, for example, may be not less than 50%, preferably not less than 60%, more preferably not less than 70%, still more preferably not less than 75% at the time of completion of the formation of the sheet-shaped cell culture. In an embodiment, the production method of the present disclosure further includes, before seeding the cells, a step of freezing the cell population and a step of thawing the frozen cell population. In this embodiment, the cell population obtained by the thawing may be seeded after subsequent proliferation of the cells. In a preferred embodiment, however, such a cell proliferation step is not included between the thawing step of the frozen cell population and the step (I). A sheet-shaped cell culture produced in such an embodiment is higher in activities such as, for example, cytokine productivity, engraftment ability, blood vessel induction ability, and tissue regeneration ability, than the sheet-shaped cell culture produced in the embodiment including the cell proliferation step between the thawing step of the frozen cell population and the step (I). Here, the expression "higher in activities" means, but is not limited to, that the activities are higher by, for example, not less than 5%, not less than 10%, not less than 20%, not less than 30%, not less than 40%, not less than 50%, not less than 60%, not less than 70%, not less than 80%, not less than 90%, not less than 100% in comparison with the corresponding activities of the comparison target sheet-shaped cell culture. In another preferred embodiment, a sheet-shaped cell culture of the present disclosure, which contains skeletal myoblasts, for example, includes the skeletal myoblasts at 60% to 99%. In further preferred embodiment, a sheet-shaped cell culture of the present disclosure, which contains myocardiac cells, for example, includes the myocardiac cells at 50% to 70%.

The sheet-shaped cell culture produced by the production method of the present disclosure may preferably a sheet-shaped cell culture for use in the treatment of a heart disease. Thus, in a preferred embodiment of the present disclosure, the cell population to be seeded includes skeletal myoblasts and fibroblast cells. In another preferred embodiment of the present disclosure, the cell population to be seeded includes myocardiac cells and vascular endothelial cells. In a further preferred embodiment of the present disclosure, the cell population to be seeded includes skeletal myoblasts, fibroblast cells, and vascular endothelial cells.

The expression "a density capable of forming a sheet-shaped cell culture without substantial proliferation" means a cell density capable of forming a sheet-shaped cell culture when cultured with a culture solution of a non-proliferation system in which growth factors are not contained substantially. This seeding density is higher than the seeding density in a method that uses a growth factor-containing culture solution, and may be equal to or higher than a density at which cells reach a confluent state. Such a density is, for example, not less than 1.0 × 10⁵ cells/cm² although not limited thereto. The upper limit of the seeding density is not restricted in particular, insofar as the formation of a cell culture is not impaired and cells do not proceed to differentiation. For example, the upper limit may be less than 3.4 × 10⁶ cells/cm². In an embodiment, the expression "a density capable of forming a sheet-shaped cell culture without substantial proliferation of cells" is equal to or higher than a density at which the cells reach a confluent state, or higher than such a confluent-state density.

The expression "a density capable of forming a sheet-shaped cell culture without substantial proliferation of cells" means 1.0 × 10⁵ to 3.4 × 10⁶ cells/cm² in an embodiment, 3.0 × 10⁵ to 3.4 × 10⁶ cells/cm² in another embodiment, 3.5 × 10⁵ to 3.4 × 10⁶ cells/cm² in a further embodiment, 1.0 × 10⁶ to 3.4 × 10⁶ cells/cm² in a still another embodiment, 3.0 × 10⁵ to 1.7 × 10⁶ cells/cm² in an even further embodiment, 3.5 × 10⁵ to 1.7 × 10⁶ cells/cm² in a still even further embodiment, and 1.0 × 10⁶ to 1.7 × 10⁶ cells/cm² in a yet even further embodiment. The above-described ranges may include both or the one of the upper limit and the lower limit insofar as the upper limit is less than 3.4 × 10⁶ cells/cm². Thus, the above-described density may be, for example, 3.0 × 10⁵ cells/cm² (inclusive) to 3.4 × 10⁶ cells/cm² (exclusive), 3.5 × 10⁵ cells/cm² (inclusive) to 3.4 × 10⁶ cells/cm² (exclusive), 1.0 × 10⁶ cells/cm² (inclusive) to 3.4 × 10⁶ cells/cm² (exclusive), 1.0 × 10⁶ cells/cm² (exclusive) to 3.4 × 10⁶ cells/cm² (exclusive), or 1.0 × 10⁶ cells/cm² (exclusive) to 1.7 × 10⁶ cells/cm² (inclusive).

In step (II), the step of making the seeded cells into a sheet may be performed by a known arbitrary technique and known arbitrary conditions. Non-limited examples of such a technique are described in, for example, Patent Literature 1, WO 2014/185517, etc. It is considered that making the cells into a sheet is achieved by cells mutually adhering via the intercellular adhesion mechanisms of adhesion molecules, the extracellular matrix, etc. Thus, the step of making the seeded cells into a sheet can be achieved by, for example, culturing the cells under conditions for forming intercellular adhesion. Such conditions may be any conditions whereby intercellular adhesion can be formed, but usually intercellular adhesion can be formed under conditions similar to common cell culture conditions. Examples of such conditions include culture at approximately 37°C and a CO₂ concentration of 5%. The culture may be performed under ordinary pressure (under atmospheric pressure or under non-pressurization). The culture may be performed in a container with an arbitrary size and an arbitrary shape. The size and the shape of the sheet-shaped cell culture can be arbitrarily adjusted by regulating the size and the shape of the cell attachment surface of the culture container, alternatively placing a frame with a desired size and a desired shape on the cell attachment surface of the culture container and culturing the cells in the culture container, or the like. In the present disclosure, the culture for making the seeded cells into a sheet may be referred to as "sheet-making culture".

In an embodiment of the present disclosure, in the case where the seeded cells include myoblast cells, the culture of the cells is performed within a prescribed period, preferably within a period during which the myoblast cell does not transition to differentiation. Thus, in this embodiment, the myoblast cell is maintained in an undifferentiated state during the culture period. The transition of the myoblast cell to differentiation can be assessed by an arbitrary method known to those skilled in the art. For example, in the case of skeletal myoblasts, the expression of an MHC, creatine kinase (CK) activity, the multinucleation of cells, the formation of a myotube, etc. may be used as indices of differentiation. The culture period may be, for example, within approximately 48 hours, within approximately 40 hours, within approximately 36 hours, within approximately 30 hours, within approximately 26 hours, or within approximately 12 hours. In a certain embodiment, the culture period may be approximately 2 hours to approximately 36 hours, approximately 2 hours to approximately 30 hours, approximately 2 hours to approximately 26 hours, approximately 2 hours to approximately 12 hours, or the like.

Step (III) may use the detaching method of the present disclosure mentioned above.

Step (IV) may use the cleaning method of the present disclosure mentioned above.

Step (V) may use the preservation method of the present disclosure mentioned above.

Step (VI) may use the shaking method of the present disclosure mentioned above.

### Examples

The present disclosure will now be described in more detail with reference to the following examples; however, these show certain specific examples of the present disclosure, and the present disclosure is not limited to these.

### <Production of a sheet-shaped cell culture>

### Example 1: changing of a culture medium

Skeletal myoblasts (including fibroblast cells) prepared from a human skeletal muscle by a usual method were used to prepare a sheet-shaped cell culture. A cell mixture of human skeletal myoblasts and human fibroblast cells suspended in a 20% human serum-containing DMEM/F12 culture medium (Thermo Fisher Scientific Inc.) was seeded in a temperature-responsive culture dish (UpCell^{(R)} 12 Holes Multi-Well, CellSeed Inc.) in such a manner that the density was 3.7 × 10⁶/well, and sheet-making culture was performed at 37°C, at a CO₂ concentration of 5%, and for 2 to 12 hours. After the sheet-making culture, the culture medium was removed from the culture dish, and 700 µL of cooled HBSS(+) (Thermo Fisher Scientific Inc.) was added and removed. This was repeated; the test object was allowed to stand still for 10 minutes after the first or second round of buffer solution addition, and then pipetting was performed quietly to detach the sheet-shaped cell culture from the culture dish completely.

In the case where there was a twist or an overlap in the detached sheet-shaped cell culture, shaking manipulation was performed for extending, and the twist or the overlap was removed. As a result, a sheet-shaped cell culture free of shrinkage or adhesion was obtained. The progress of new intercellular adhesion was suppressed successfully by removing the culture medium and substituting it with HBSS(+), and performing shaking manipulation. In the case where the culture medium was not removed or in the case where a twist or an overlap of the sheet-shaped cell culture was left as it was (in the case where there was no shaking manipulation), a twist or shrinkage, and adhesion or sticking resulting from it occurred in the sheet-shaped cell culture. The results are shown in Fig. 1.

### Example 2: perfusion of both surfaces

The HBSS(+) was removed from the culture dish containing the detached sheet-shaped cell culture obtained in example 1, and newly HBSS(+) at normal temperature was added and the sheet-shaped cell culture was soaked; thereby, both surfaces (the upper surface and the lower surface) were perfused. The step of such removal and addition was performed once more. The HBSS(+) was removed, and then 1 mL of new HBSS(+) was added (removal and addition were repeated 3 times in total); then, the test object was allowed to stand still at room temperature.

In the case where there was a twist or an overlap in the detached sheet-shaped cell culture, shaking manipulation was performed for extending, and the twist or the overlap was removed. As a result, a sheet-shaped cell culture free of shrinkage or adhesion was obtained. The progress of new intercellular adhesion was suppressed successfully by perfusing both surfaces of the sheet-shaped cell culture. In the case where a twist or an overlap of the sheet-shaped cell culture was left as it was (in the case where there was no perfusion manipulation), shrinkage and adhesion occurred in the sheet-shaped cell culture. The results are shown in Fig. 2.

### Example 3: preservation of the sheet-shaped cell culture

The sheet-shaped cell culture obtained in example 2 was subsequently preserved in HBSS(+) at normal temperature for 10 hours to 5 days. An enough amount of HBSS(+) for the sheet-shaped cell culture to be sufficiently soaked in the culture dish was used.

In the case where there was a twist or an overlap in the preserved sheet-shaped cell culture, shaking manipulation was performed for extending, and the twist or the overlap was removed. As a result, a sheet-shaped cell culture free of shrinkage or adhesion was obtained. The progress of new intercellular adhesion was suppressed successfully by preserving the sheet-shaped cell culture at normal temperature. In the case where a twist or an overlap of the sheet-shaped cell culture was left as it was (in the case where there was no shaking manipulation), shrinkage and adhesion occurred in the sheet-shaped cell culture. The results are shown in Fig. 3.

## Claims

1. A method for suppressing shrinkage when detaching a sheet-shaped cell culture formed on a culture substrate, the method comprising:
(a) soaking, in a low nutrient isotonic solution, an upper surface of the sheet-shaped cell culture of which at least part is adhered to the culture substrate; and
(b) detaching the sheet-shaped cell culture in the low nutrient isotonic solution.

2. The method according to claim 1, wherein the culture substrate is covered with a serum.

3. The method according to claim 1 or 2, wherein the culture substrate is covered with a temperature-responsive material.

4. The method according to any one of claims 1 to 3, wherein the sheet-shaped cell culture contains a myoblast cell.

5. The method according to any one of claims 1 to 4, wherein the low nutrient isotonic solution is at 2 to 8°C.

6. The method according to any one of claims 1 to 5, wherein the low nutrient isotonic solution is Hanks' balanced salt solution.

7. The method according to any one of claims 1 to 6, wherein a production step-derived impurity remaining in the sheet-shaped cell culture is removed by the soaking.

8. The method according to any one of claims 1 to 7, further comprising: extending the detached sheet-shaped cell culture.

9. The method according to claim 8, wherein the extending of the sheet-shaped cell culture is performed by shaking a container containing the low nutrient isotonic solution in which the sheet-shaped cell culture is soaked.

10. The method according to any one of claims 1 to 9, wherein the shrinkage is caused by intercellular adhesion in the sheet-shaped cell culture.

11. The method according to any one of claims 1 to 10, wherein the extending makes rotation with a middle of a container as a center.
